# EUROPÄISCHE PATENTSCHRIFT

(11) **EP 3 008 349 B1**
(45) Veröffentlichungstag und Bekanntmachung des Hinweises auf die Patenterteilung: **05.10.2022**
(21) Anmeldenummer: 14739361.5
(22) Anmeldetag: 13.06.2014
(51) Int. Cl.: A61B 90/00, F16B 2/12, A61B 34/20, A61B 17/00

(54) **ADAPTER ZUR AUFNAHME EINES MEDIZINTECHNISCHEN GERÄTS SOWIE EINES LAGEERFASSUNGSSYSTEMS**
ADAPTER FOR RECEIVING A MEDICAL TECHNICAL DEVICE AND A POSITION DETECTING SYSTEM
ADAPTATEUR DESTINÉ À RECEVOIR UN APPAREIL MÉDICAL ET UN SYSTÈME DE DÉTECTION DE POSITION

(30) Priorität: 13.06.2013 DE 102013211055
(43) Veröffentlichungstag der Anmeldung: 20.04.2016
(73) Patentinhaber: Stryker European Operations Limited, Carrigtwohill, Co. Cork T45 HX08 (IE)
(72) Erfinder: REUTTER, Andreas, 10439 Berlin (DE); ÖZBEK, Christopher, 10823 Berlin (DE); KOSMECKI, Bartosz, 10965 Berlin (DE)
(74) Vertreter: Röthinger, Rainer
(86) Internationale Anmeldenummer: PCT/EP2014/062424
(87) Internationale Veröffentlichungsnummer: WO 2014/198922

(56) Entgegenhaltungen:
- DE-U1-202006 019 649
- US-A- 3 581 354
- US-A- 4 867 404
- US-A1- 2005 041 966
- US-A1- 2009 321 599

## Beschreibung

Die Erfindung betrifft einen Adapter zur Aufnahme eines medizintechnischen Geräts sowie eines Lageerfassungssystems.

Aus der Medizintechnik sind klinische Navigationssysteme bekannt, mit denen sich z. B. während einer Operation die räumliche Position eines Instrumentes (z. B. eines Endoskops) bestimmen lässt, um diese lagerichtig in Bilddarstellungen (z. B. in Form einer CT-Aufnahme) eines Operationsgebietes einblenden zu können. Die lagerichtige Einblendung erfordert eine Transformation der mittels des Lageerfassungssystems bestimmten räumlichen Position des Instrumentes in das Koordinatensystem der Darstellung des Operationsgebietes. Als Lageerfassungssysteme werden insbesondere optische Messsysteme genutzt, die mit Hilfe mehrerer Kameras die Position von Trackern, bestehend aus mehreren fest verbundenen, meist kugelförmigen Markerelementen, erfassen. Alternativ kommen auch elektromagnetische Messsysteme zum Einsatz, bei denen die Position von Sensorspulen in einem von einem Feldgenerator erzeugten elektromagnetischen Feld gemessen wird.

Dokument US 2009/321599 A1 offenbart einen Adapter (30) zur Aufnahme eines Befestigungsschafts (20), umfassend: a. eine Klammer (31) und eine Klemmbacke (33), wobei die Klammer (31) eine Aufnahmeöffnung (313) mit einer Längserstreckungsrichtung aufweist, und wobei die Klemmbacke (33) beweglich in der Aufnahmeöffnung (313) der Klammer (31) gelagert ist; b. eine Feder (35), welche zwischen der Klemmbacke (33) und einem Positionierring (341), der an einer Unterseite der Klammer (31) festgelegt ist, angeordnet ist, wobei die Rückstellkraft der Feder (35) in der Längserstreckungsrichtung der Aufnahmeöffnung (313) der Klammer (31) verläuft und auf die Klemmbacke (33) wirkt; c. eine erste Einführöffnung (311), die die Klammer (31) komplett durchdringt, und eine zweite Einführöffnung (312), die die Klemmbacke (33) komplett durchdringt, beide zur Einführung des Befestigungsschafts (20), wobei die erste und zweite Einführöffnung (311, 312) quer zur Rückstellkraft der Feder (35) ausgebildet und durch eine Krafteinwirkung entgegen der Rückstellkraft der Feder (35) zur Deckung bringbar sind, wodurch ein Aufnahmezustand des Adapters (30) vorliegt, und wobei ohne die Krafteinwirkung entgegen der Rückstellkraft der Feder (35) ein Verklemmungszustand des Adapters (30) vorliegt; d. eine Sperre (36), welche mit der Oberseite der Klemmbacke (33) über eine Nockenkupplung (332/361/362) verbunden ist, und welche eine Befestigungswelle (363) aufweist; e. ein Griff (37), welcher über die Befestigungswelle mit der Sperre (36) verbunden ist, so dass sich mittels einer Drehbewegung des Griffs (37) die Sperre (36) drehen lässt, und so die Nockenkupplung (332/361/362) die Klemmbacke (33) entgegen der Richtung der Rückstellkraft der Feder (35) drückt, bis dadurch ein Aufnahmezustand des Adapters (30) erreicht wird; und f. ein Haltebügel (34) zum Befestigen eines Duschkopfs (40), einer Seifenschale (50) oder dergleichen.

Dokument US 2009/321599 A1 offenbart außerdem einen Adapter (62) zur Aufnahme eines Befestigungsschafts (61), umfassend: a. eine Klammer (63) und eine Klemmbacke (66), wobei die Klammer (63) eine Aufnahmeöffnung mit einer Längserstreckungsrichtung aufweist, und wobei die Klemmbacke (66) beweglich in der Aufnahmeöffnung der Klammer (63) gelagert ist; b. eine erste Einführöffnung (631), die die Klammer (63) komplett durchdringt, und eine zweite Einführöffnung (661), die die Klemmbacke (66) komplett durchdringt, beide zur Einführung des Befestigungsschafts (61), wobei die erste und zweite Einführöffnung (631, 661) quer zur Längserstreckungsrichtung ausgebildet sind; c. ein Gewindekopf, welcher an der Oberseite der Klemmbacke (66) festgelegt ist, und welcher an seiner Außenhülle ein Gewinde aufweist; d. ein Griff (65), welcher über ein Gegengewinde (632) mit dem Gewinde des Gewindekopfs verbunden ist, so dass sich mittels einer Drehbewegung des Griffs (65) das Gewinde des Gewindekopfs in Längserstreckungsrichtung in den Griff (65) bewegen lässt, bis dadurch ein bewegungssicherer Verklemmungszustand des Adapters (62) erreicht wird; und e. ein Haltebügel (64) zum Befestigen eines Duschkopfs, einer Seifenschale oder dergleichen.

Die US 3 581 354 A offenbart eine Klammer zur Montage eines Instruments, wie beispielsweise einer Messuhr oder einem Testindikator an einem Stützgestell. Die Klammer ist so aufgebaut, dass ein Testindikator mit verschiedene Größen und Typen angebracht werden kann. Ein Wechsel zwischen Aufnahme- und Verklemmzustand wird mittels einer Klemmschraube erreicht.

In der US 4 867 404 A wird eine flexible Halterung und Klemmvorrichtung zum einstellbaren Halten von Zystoskopen oder andere endoskopische Instrumente und Retraktoren oder dergleichen offenbart. Die Halterung dient der Befestigung neben oder auf einem Untersuchungstisch oder dergleichen und ist mit einer Klemmvorrichtung ausgestattet, die den Betrieb von verschiedenen großen Instrumentenwellen ermöglicht. Die Spannvorrichtung ist mit einem vertikal verstellbaren federvorgespannten C-förmigen, seitlich offenen Bereich ausgestattet, die in eine normalerweise offene Position gedrängt wird.

Die DE 20 2006 019649 U1 offenbart eine Führungsrohr-Fixiervorrichtung zum Befestigen oder Fixieren eines Führungsrohrs bei einer oder an einer Struktur zum Zuführen einer Substanz an einen Ort, wobei die Führungsrohr-Fixiervorrichtung ein Befestigungselement, ein an dem Befestigungselement befestigtes Gelenk und eine an dem Gelenk befestigte Führungsrohr-Haltevorrichtung umfasst, wobei die Führungsrohr-Haltevorrichtung ein Führungsrohr führen kann, durch das eine Kanüle oder eine Nadel geführt oder eine Substanz an eine gewünschte Stelle oder an einen gewünschten Ort direkt zugeführt werden kann.

Die US 2005/041966 A1 offenbart eine Klammer zur Befestigung von fotografischer Ausrüstung an einem Ständer.

Das von der vorliegenden Erfindung zu lösende Problem besteht darin, einen Adapter zur Aufnahme eines medizintechnischen Geräts sowie eines Lageerfassungssystems bzw. einer Komponente eines Lageerfassungssystems bereitzustellen, der es ermöglicht auf einfache Art und Weise eine Verbindung zwischen einem Lageerfassungssystem und einem medizintechnischen Gerät bereitzustellen und eine variable Einstellung und Kombinierbarkeit der medizintechnischen Geräte mit einem Lageerfassungssystem eines klinischen Navigationssystems gestattet.

Diese Probleme werden durch den Adapter mit den Merkmalen gemäß Anspruch 1 gelöst. Weiterbildungen der Erfindung sind in den abhängigen Ansprüchen angegeben.

Danach wird ein Adapter zur Aufnahme eines medizintechnischen Geräts sowie eines Lageerfassungssystems bereitgestellt, der ein erstes Halteelement, ein zweites Haltelement, eine eine Rückstellkraft auf das zweite Halteelement ausübende Feder, welche wirkverbunden mit einer Unterseite des zweiten Halteelements ausgebildet ist, und ein Befestigungselement zum Befestigen des Lageerfassungssystems aufweist.

Dabei weist das erste Halteelement eine Aufnahmeöffnung mit einer Längserstreckungsrichtung auf, die dazu ausgebildet ist, das zweite Halteelement innerhalb des ersten Halteelements aufzunehmen, wobei die Längserstreckungsrichtung der Aufnahmeöffnung des ersten Haltelements parallel zur Richtung der Rückstellkraft der Feder ausgebildet ist.

Erfindungsgemäß ist das zweite Halteelement beweglich in der Aufnahmeöffnung des ersten Halteelements gelagert und durch eine Krafteinwirkung entgegen der Rückstellkraft der Feder in eine Richtung entgegen der Rückstellkraft der Feder derart bewegbar, dass ein Aufnahmezustand des Adapters erreicht wird. In dem Aufnahmezustand ist das medizintechnische Gerät in den Adapter einführbar.

Bei Abwesenheit der Krafteinwirkung entgegen der Rückstellkraft der Feder vollzieht das zweite Haltelement mittels der Rückstellkraft der Feder eine Bewegung in Richtung der Rückstellkraft. Dadurch liegt ein Verklemmungszustand des Adapters vor, in dem das in den Adapter eingeführte medizintechnische Gerät zwischen dem ersten Haltelement und zweiten Haltelement verklemmt ist.

Dabei weist das erste Halteelement eine erste Einführöffnung, zur Einführung des medizintechnischen Geräts, und das zweite Haltelement eine zweite Einführöffnung, zur Einführung des medizintechnischen Geräts, auf, wobei die erste und zweite Einführöffnung quer zur Rückstellkraft der Feder ausgebildet sind und die erste Einführöffnung bzw. die zweite Einführöffnung das erste Haltelement bzw. das zweite Halteelement komplett durchdringt. Dabei ist die erste Einfuhröffnung mit der zweiten Einfuhröffnung durch die Krafteinwirkung entgegen der Rückstellkraft der Feder zur Deckung bringbar und der Aufnahmezustand des Adapters erreicht. Bei Abwesenheit der Krafteinwirkung liegt durch die Rückstellkraft der Feder der Verklemmungszustand des Adapters vor.

An dem zweiten Halteelement ist ein erstes Verstellelement angeordnet, welches an seiner Außenhülle ein Gewinde aufweist. Das erste Verstellelement ist mittels des Gewindes mit einem zweiten Verstellelement, welches eine Aufnahmeöffnung mit einem Gegengewinde zur Aufnahme des ersten Verstellelements aufweist, derart wirkverbunden, dass mittels einer Drehbewegung des zweiten Verstellelement in Verklemmungsrichtung (also der Drehrichtung des zweiten Verklemmungselements, die eine Bewegung des zweiten Halteelements in Richtung der Rückstellkraft der Feder ermöglicht) das erste Verstellelement und somit das zweite Halteelement in Richtung der Rückstellkraft der Feder bewegbar ist. Dadurch liegt ein bewegungssicherer Verklemmungszustand des Adapters vor, d. h. das medizintechnische Gerät ist ortsfest an dem Adapter festgelegt.

In einer zweiten Ausführungsform weist das erste Halteelement ein erstes Klammerelement und das zweite Haltelement ein sich durch die Aufnahmeöffnung nach außen hin erstreckendes zweites Klammerelement auf. Dabei ist das zweite Haltelement durch die Krafteinwirkung entgegen der Rückstellkraft der Feder derart bewegbar, dass der Abstand zwischen dem ersten Klammerelement und dem zweiten Klammerelement vergrößert wird und dadurch der Aufnahmezustand des Adapters vorliegt. Bei Abwesenheit der Krafteinwirkung durch die Rückstellkraft der Feder liegt der Verklemmungszustand des Adapters vor.

Weiterhin kann der bewegungssichere Verklemmungszustand des Adapters durch eine Drehbewegung des zweiten Verstellelements entgegen der Verklemmungsrichtung aufgelöst werden.

Weiterhin kann der Verklemmungszustand des Adapters durch eine Krafteinwirkung entgegen der Rückstellkraft der Feder aufgehoben werden und das medizintechnische Gerät anschließend in diesem Zustand aus dem Adapter entfernt werden.

In einer weiteren Ausführungsform ist das Befestigungselement an dem zweiten Halteelements festgelegt und das Lageerfassungssystem weist ein Befestigungsgegenstück auf, welches das Befestigungselement in Eingriff nehmen kann.

Dabei kann vorgesehen sein, dass das Befestigungselement eine Aushöhlung mit einem Gewinde und das Befestigungsgegenstück eine Aufnahmeöffnung mit einem identischen Gewinde aufweist, wobei die Aushöhlung des Befestigungselements und die Aufnahmeöffnung des Befestigungsgegenstücks in Richtung der Rückstellkraft der Feder ausgerichtet sind. Weiterhin sind das Befestigungselement und das Befestigungsgegenstück derart ausgebildet, dass mittels einer Schraube und einer Mutter ein Verschlusszustand des Lageerfassungssystems mit dem Adapter durch eine kraftschlüssige Verbindung des Befestigungselements, des Befestigungsgegenstücks, der Schraube und der Mutter über eine Drehbewegung der Mutter in Verschlussrichtung hergestellt werden kann.

Alternativ oder zusätzlich können das Befestigungselement und das Befestigungsgegenstück an ihren gegenseitigen Kontaktflächen ineinander eingreifende Strukturierungen, insbesondere ineinander eingreifende Zahnstrukturen, aufweisen, so dass im Verschlusszustand ein form- und kraftschlüssiger Verschluss des Lageerfassungssystems mit dem Adapter vorliegt.

In einer weiteren Ausführungsform kann vorgesehen sein, dass in einem nicht verschlossenen Zustand das Lageerfassungssystem drehbar gelagert ist und somit die räumliche Ausrichtung des Lageerfassungssystems variabel einstellbar ist. Insbesondere kann die räumliche Anordnung des Lageerfassungssystems in vorgegebenen Winkeln eingestellt werden, die durch die Größe der Strukturelemente des Befestigungselements und des Befestigungsgegenstücks festgelegt sind.

Weiterhin kann das Lageerfassungssystem drei oder mehr Markierungselemente aufweisen, die fest miteinander und mit dem Befestigungsgegenstück verbunden sind. Alternativ kann das Lageerfassungssystem aus einer Sensorspule zur elektromagnetischen Positionsmessung mit fest verbundenem Befestigungsgegenstück bestehen. Auch kann das Lageerfassungssystem aus einem Kamerasystem aus einer oder mehreren Kameras mit fest verbundenem Befestigungsgegenstück aufgebaut sein.

Bei den medizintechnischen Geräten kann es sich beispielsweise um chirurgische Instrumente handeln wie z. B. ein Absaugrohr oder ein Pointer zum Registrieren von Oberflächenpunkten.

Der erfindungsgemäße Adapter besteht bevorzugt aus Metall, insbesondere aus einer Titanlegierung. Allerdings ist bei Nutzung elektromagnetischer Messverfahren die Verwendung von Kunststoff vorteilhaft.

Die Erfindung wird im Folgenden anhand von Ausführungsbeispielen unter Bezugnahme auf die Figuren näher erläutert. Es zeigen:

- Fig. 1: eine Ausgestaltungsform des erfindungsgemäßen Adapters zur Aufnahme eines medizintechnischen Geräts sowie eines Lageerfassungssystems in einer Explosionsansicht;
- Fig. 2: die Ausführungsform der Figur 1 in einem unverklemmten Zustand;
- Fig. 3: die Ausführungsform der Figur 1 in einem verklemmten Zustand;
- Fig. 4: einen Querschnitt der Ausführungsform der Figur 1 in einem verklemmten Zustand;
- Fig. 5: zeigt die Ausführungsform der Figur 1 mit einem Lageerfassungssystem in Form eines Trackers mit drei Markerelementen zur optischen Positionsmessung und einem Absaugrohr in einem verklemmten Zustand;
- Fig. 6: zeigt die Ausführungsform der Figur 1 mit einem Lageerfassungssystem in Form eines Trackers mit drei Markerelementen zur optischen Positionsmessung und einem Pointer zum Registrieren von Oberflächenpunkten;
- Fig. 7: zeigt eine zweite erfindungsgemäße Ausführungsform im Querschnitt; und
- Fig. 8: zeigt die zweite Ausführungsform der Figur 7 mit einem Lageerfassungssystem in einem verklemmten Zustand;

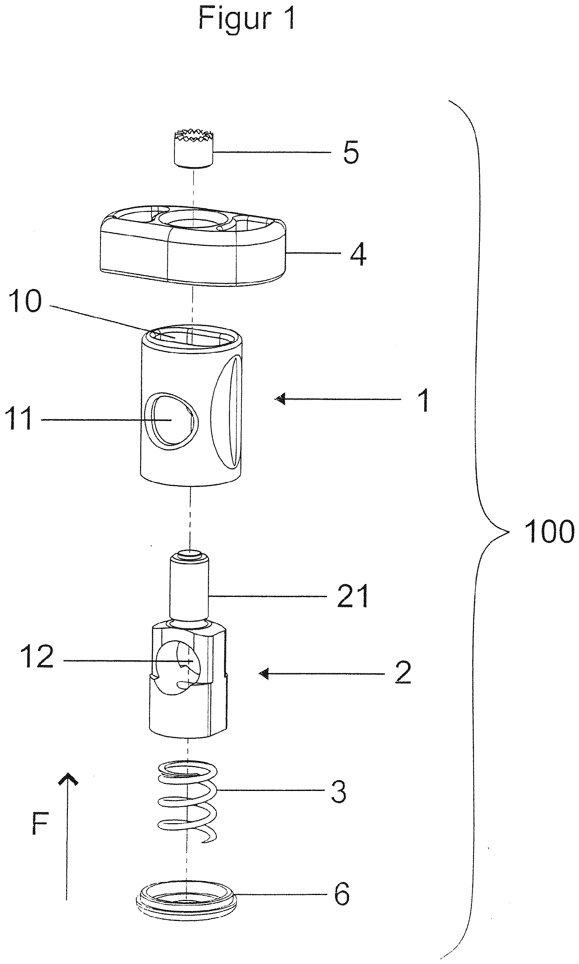

Die in Fig. 1 in einer Explosionsansicht dargestellte erste Ausführungsform des Adapters 100 weist ein erstes Halteelement 1 auf, welches eine in Längserstreckungsrichtung erstreckende Aufnahmeöffnung 10 und eine erste Einführöffnung 1 1 aufweist Die Aufnahmeöffnung 10 erstreckt sich in Richtung der Rückstellkraft der Feder 3 komplett durch das erste Halteelement 1 und ist derart ausgebildet, dass ein zweites Halteelement 2 in dem ersten Haltelement 1 aufgenommen werden kann.

Weiterhin ist die erste Einführöffnung 1 1 des ersten Halteelements 1 zur Einführung eines medizintechnischen Geräts (hier nicht dargestellt) derart ausgebildet, dass sich die Einführöffnung 1 1 quer zur Richtung der Rückstellkraft der Feder 3 durch das komplette erste Haltelement 1 erstreckt.

Zusätzlich weist der Adapter 100 ein zweites Halteelement 2 mit einer zweiten Einführöffnung 12 auf. Die zweite Einführöffnung 12 ist derart ausgebildet, dass sie sich quer zur Richtung der Rückstellkraft der Feder 3 komplett durch die zweite Haltevorrichtung 2 erstreckt. Dabei ist die Feder 3 des Adapters 100 unterhalb des zweiten Halteelements 2 angeordnet, wobei die Feder 3 derart angeordnet ist, dass sie mit der Unterseite des zweiten Halteelements 2 in Wirkverbindung tritt.

Das erste Halteelement 1 des Adapters 100 ist an dem Bodenstück 6, beispielsweise mittels einer Klebeverbindung oder Verschwei ßen, festgelegt. Dabei ist die Feder 3 innerhalb des ersten Halteelements 1 angeordnet. Weiterhin ist das zweite Haltelement 2 oberhalb der Feder 3 (und ebenfalls innerhalb des ersten Haltelements 1 ) angeordnet, wobei das das zweite Halteelement innerhalb des ersten Halteelements 1 derart beweglich angeordnet ist, dass das Halteelement 2 durch eine Krafteinwirkung entgegen der Richtung der Rückstellkraft der Feder 3 - wenn die Krafteinwirkung größer ist als die Rückstellkraft der Feder - entgegen der Richtung der Rückstellkraft der Feder bewegbar ist bzw. in Abwesenheit einer Krafteinwirkung auf die Feder 3, durch die Rückstellkraft der Feder 3 in Richtung der Rückstellkraft der Feder 3 bewegbar ist.

Dabei ist bei dem erfindungsgemäßen Adapter 100 vorgesehen, dass die erste Einführöffnung 11 und die zweite Einfuhröffnung 12 des ersten Halteelements 1 und des zweiten Halteelements 2 durch die Einwirkung einer Kraft entgegen der Rückstellkraft der Feder 3 zur Deckung gebracht werden können. Durch die Einwirkung einer derartigen Kraft liegt beim Erreichen der Deckungsgleichheit der Einfuhröffnungen 11 und 12 der Aufnahmezustand des Adapters 100 für ein medizintechnisches Gerät vor. Das medizintechnische Gerät kann in diesem Aufnahmezustand durch die beiden Einführöffnungen 11 und 12 in den Adapter 100 eingeführt werden.

In Abwesenheit der Krafteinwirkung auf die Feder 3 bewirkt die Rückstellkraft der gespannten Feder 3 eine Bewegung des zweiten Haltelements 2 in Richtung der Rückstellkraft der Feder 3. Dadurch wird ein Verklemmungszustand des Adapters 100 erzielt, in dem ein in den Adapter 100 eingeführtes medizintechnisches Gerät zwischen dem ersten Haltelement 1 und dem zweiten Halteelement 2 verklemmt wird.

Der erfindungsgemäße Adapter 100 weist des Weiteren ein erstes Verstellelement 21 auf, welches mit einem Gewinde versehen ist und an dem zweiten Haltelement 2 angeordnet ist, und ein zweites Verstellelement 4, welches eine Aufnahmeöffnung mit einem Gegengewinde aufweist. Dabei ist das zweite Verstellelement 4 über die Aufnahmeöffnung derart mit dem ersten Verstellelement 21 wirkverbunden, dass bei einer Drehbewegung in Verschlussrichtung (einer Drehbewegung, bei der sich das zweite Verstellelement 4 in Richtung entgegen der Rückstellkraft der Feder 3 bewegt) das erste Verstellelement 21 und somit das zweite Haltelement 2 in Richtung der Rückstellkraft der Feder 3 bewegt wird. Dadurch wird ein bewegungssicherer Verklemmungszustand des Adapters 100 erzielt, in dem ein in den Adapter 100 eingeführtes medizintechnisches Gerät zwischen dem ersten Haltelement 1 und dem zweiten Halteelement 2 verklemmt wird. Unter bewegungssicher ist zu verstehen, dass das medizintechnische Gerät nicht mehr in Einführrichtung durch die Einführöffnungen 11 und 12 bewegbar ist, also ortsfest mit dem Adapter 100 verbunden ist.

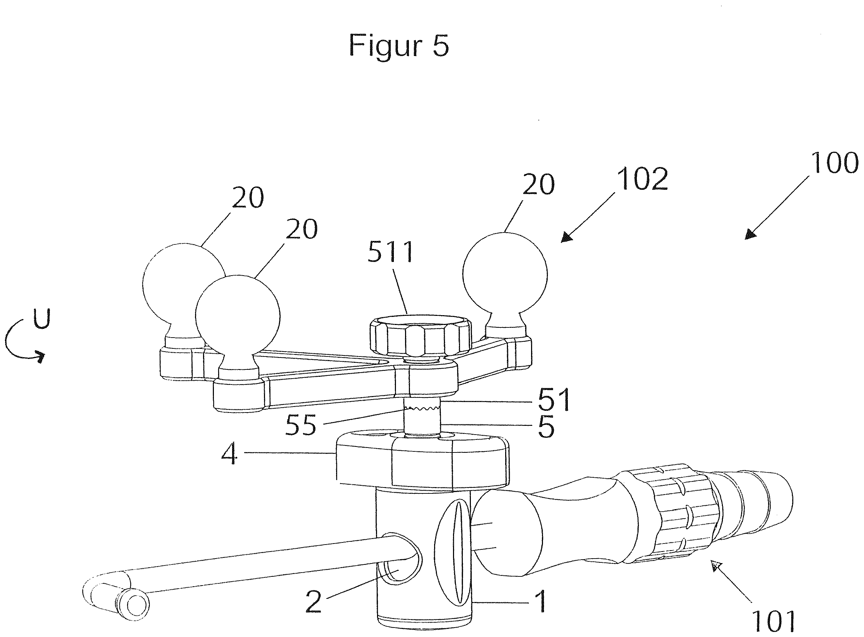

Weiterhin weist der erfindungsgemäße Adapter 100 noch ein Befestigungselement 5 zur Befestigung eines Lageerfassungssystems auf, welches an der Oberseite des Verstellelements 21 , beispielsweise durch Verschwei ßen oder Verkleben, festgelegt ist. Hinsichtlich der Funktionsweise des Befestigungselements 5 zum Befestigen des Lageerfassungssystems wird auf die Erläuterungen der Fig. 5 verwiesen.

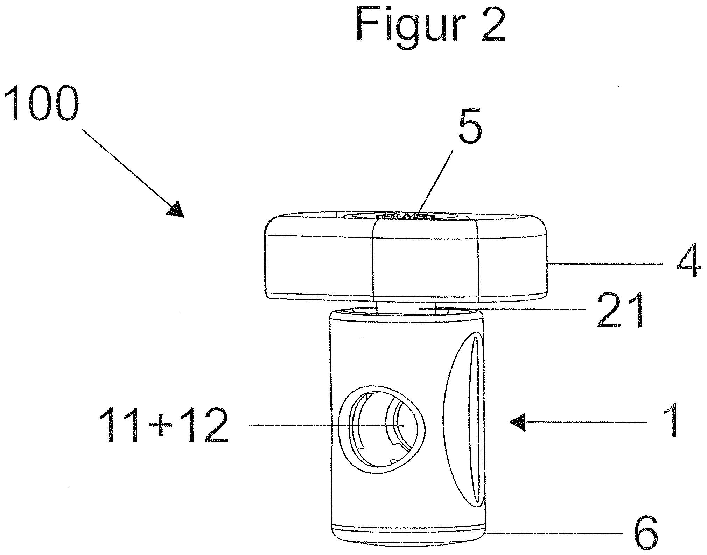

Die Fig. 2 zeigt die Ausführungsform der Figur 1 in einem Aufnahmezustand, also einem nicht verklemmten Zustand. Dabei befindet sich das zweite Verstellelement 4 in einem geöffneten Zustand und die beiden Einführöffnungen 1 1 und 12 sind durch eine (hier nicht dargestellte) mechanische Krafteinwirkung, wie beispielsweise durch Zusammendrücken zur Deckung gebracht. Ein medizintechnisches Gerät kann nun durch die beiden Einführöffnungen 1 1 und 1 2 in den Adapter 1 00 eingeführt werden.

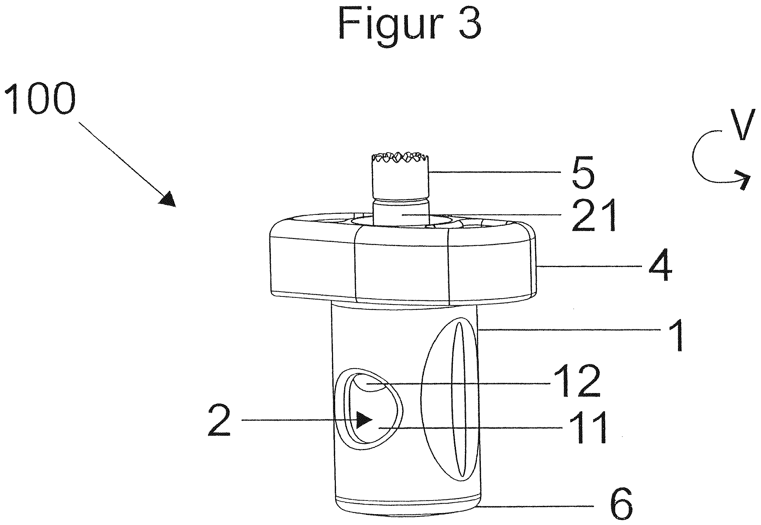

Fig. 3 zeigt Ausführungsform der Figur 1 in einem bewegungssicheren Verklemmungszustand. Dabei wurde das zweite Verstellelement 4 mittels einer Drehbewegung in Verschlussrichtung bis zum Erreichen des bewegungssicheren Verklemmungszustands gedreht. Dabei wurde das zweite Halteelement 2 in Richtung der Rückstellkraft der Feder 3 bewegt. Ein medizintechnisches Gerät, welches im Aufnahmezustand der Fig. 2 in den Adapter 1 00 eingeführt wurde, erfährt bei Erreichen des in Fig. 3 dargestellten bewegungssicheren Verklemmungszustands eine bewegungssichere Verklemmung und verbleibt somit ortsfest. Aus Gründen der Übersichtlichkeit wurde das medizintechnische Gerät in dieser Figur nicht dargestellt.

Der bewegungssichere Verklemmungszustand des medizintechnischen Geräts wird analog zu der Darstellung der Fig. 3 durch eine Verklemmung des medizintechnischen Geräts zwischen dem ersten Haltelement 1 und dem zweiten Haltelement 2 aufgrund der Bewegung des zweiten Haltelements 2 in Richtung der Rückstellkraft der Feder 3 durch der Drehbewegung des zweiten Verstellungselements 4 in Verschlussrichtung bereitgestellt.

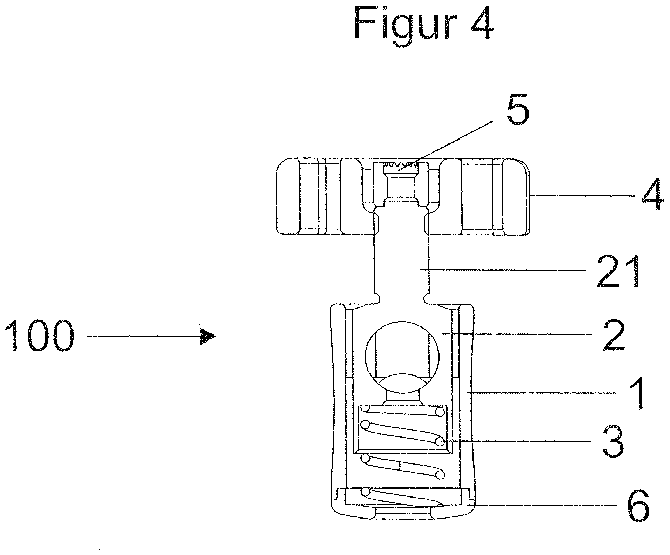

Die Fig. 4 zeigt einen Querschnitt des ersten erfindungsgemäßen Adapters in einem Verklemmungszustand. Der Verklemmungszustand wird hierbei durch die Rückstellkraft der Feder 3 erreicht, die das zweite Haltelement 2 in Richtung der Rückstellkraft der Feder 3 bewegt. Dadurch wird ein Verklemmungszustand durch Verklemmen des (hier aus Gründen der Übersichtlichkeit nicht dargestellten) medizintechnischen Geräts zwischen dem ersten Haltelement 1 und dem zweiten Haltelement 2 erzielt. Durch eine Drehbewegung des Verstellelements 4 in Verschlussrichtung kann dann analog zu der Beschreibung der Figur 3 ein bewegungssicherer Verklemmungszustand erreicht werden.

Die Figur 5 zeigt einen erfindungsgemäßen Adapter mit einem medizintechnischen Gerät (in diesem Fall ein Absaugrohr) und eines Lageerfassungssystems in Form eines Trackers zur optischen Positionsmessung mit drei Markierungselementen 20 in einem Verklemmungszustand. Hinsichtlich des Verklemmungszustandes des medizintechnischen Geräts wird auf die Beschreibungen der Figuren 2 bis 4 verwiesen.

Der erfindungsgemäße Adapter weist ein Befestigungselement 5 und das Lageerfassungssystem 102 ein Befestigungsgegenstück 51 auf, die beide an ihren Kontaktflächen über ineinandergreifende Strukturierungen 55 (hier ineinandergreifende Zähne) verfügen. Dabei wird ein Verschlusszustand des Lageerfassungssystems mit dem erfindungsgemäßen Adapter über ein Zusammenwirken des Befestigungselements 5, des Befestigungsgegenstücks 51 der Mutter 51 1 und einer hier nicht dargestellten Schraube erreicht.

Das Befestigungselement 5 weist dabei eine Aushöhlung mit einem Gewinde auf und das Befestigungsgegenstück 51 eine Aufnahmeöffnung mit einem identischen Gewinde. Das Befestigungselement 5 wird mittels einer zu dem Gewinde passenden Schraube mit dem Befestigungselement 51 verbunden und mittels der Mutter 51 1 durch eine Drehbewegung in Verschlussrichtung U in einem Verschlusszustand gebracht. Durch das Ineinandergreifen der Strukturflächen 55 an den gegenseitigen Kontaktflächen des Befestigungselements 5 und des Befestigungsgegenstücks 51 wird somit eine kraftschlüssige und formschlüssige Verbindung erreicht.

Weiterhin ist es bei dem erfindungsgemäßen Adapter vorgesehen, dass das Lageerfassungssystem 102 drehbar gelagert ist. Sofern noch kein vollständiger Verschlusszustand durch die Drehbewegung der Mutter 51 1 in Endstellung der Verschlussrichtung erreicht ist, kann die räumliche Anordnung des Lageerfassungssystems variabel eingestellt werden, wobei die einstellbaren Winkel durch die ineinandergreifenden Strukturelemente 55 des Befestigungselements 5 und des Befestigungsgegenstücks 51 vorbestimmt sind.

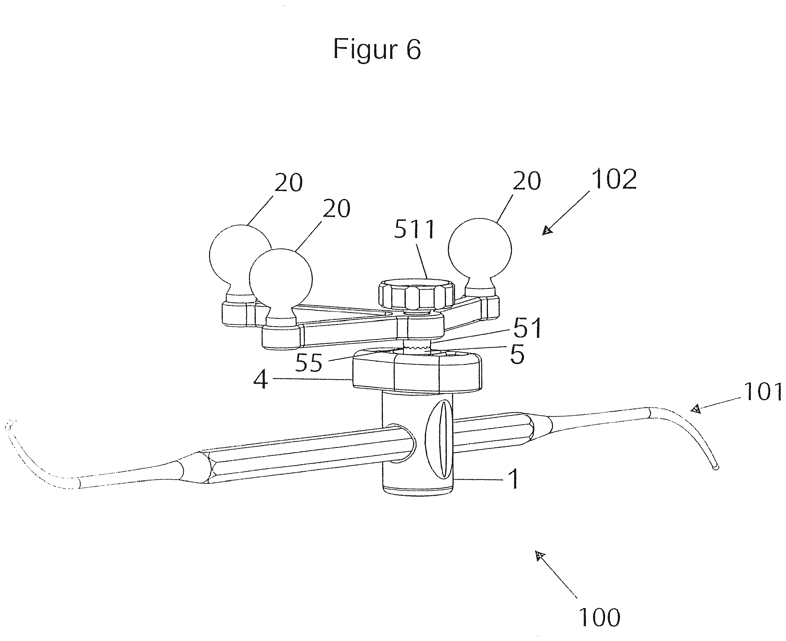

Die Figur 6 zeigt einen erfindungsgemäßen Adapter in einer ersten Ausführungsform mit einem medizintechnischen Gerät (in diesem Fall ein Pointer) und einem Lageerfassungssystem in Form eines Trackers mit drei Markierungselementen in einem Verklemmungszustand. Hinsichtlich der weiteren Beschreibungen wird auf die die Beschreibungen der Figuren 2 bis 5 verwiesen.

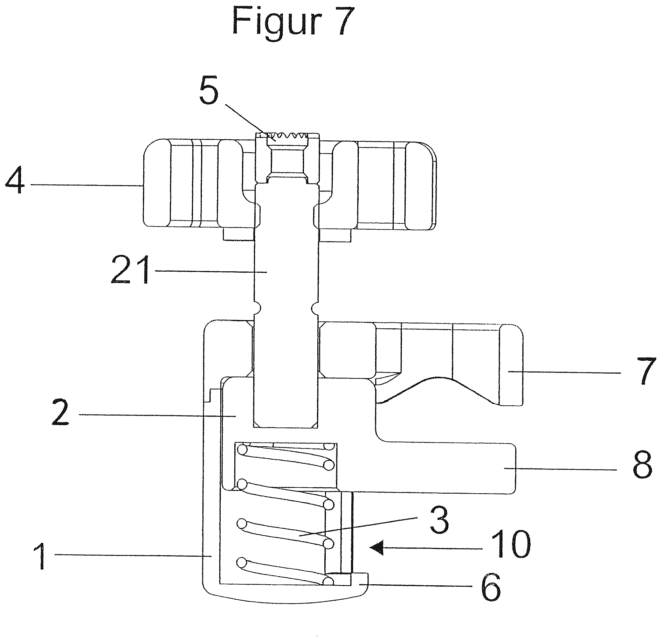

Die Figur 7 zeigt ein zweites Ausführungsbeispiel eines erfindungsgemäßen Adapters, der vergleichbar zu dem ersten Ausführungsbeispiel ein erstes Halteelement 1 und ein zweites Halteelement 2, welches bewegbar innerhalb des ersten Halteelements 1 angeordnet ist und eine Feder 3 aufweist. Der Aufnahmezustand für ein medizintechnisches Gerät wird auch in diesem Ausführungsbeispiel vergleichbar den Ausführungsbeispielen der Figuren 2 bis 4 durch eine Krafteinwirkung entgegen der Rückstellkraft der Feder 3 erreicht. Hinsichtlich einer ausführlichen Erläuterung des Aufnahmezustandes wird auf die Beschreibung der vorgenannten Figuren verwiesen.

Durch eine Krafteinwirkung entgegen der Rückstellkraft der Feder 3 wird der Abstand zwischen dem ersten Klammerelement 7 und dem zweiten Klammerelement 8 vergrößert und dadurch ein Aufnahmezustand erreicht. Nach Einführen des medizintechnischen Gerätes und bei Abwesenheit einer Krafteinwirkung entgegen der Richtung der Rückstellkraft der Feder 3 bewirkt die gespannte Feder 3 eine Bewegung des zweiten Halteelements 2 und dadurch des zweiten Klammerelements 8 in Richtung der Rückstellkraft der Feder 3. Dadurch wird ein Verklemmungszustand erreicht.

Durch Drehen des zweiten Verstellelements 4 in Verklemmungsrichtung wird das zweite Halteelement 2 (vergleichbar der Beschreibung der Figuren 2 bis 4, auf die explizit verwiesen wird) in Richtung der Rückstellkraft der Feder 3 verschoben. Dadurch wird ein bewegungssicherer Verklemmungszustand hergestellt.

Durch eine Bewegung des zweiten Verstellelements 4 entgegen der Verklemmungsrichtung wird der bewegungssichere Verklemmungszustand wieder aufgelöst und eine Entnahme des medizintechnischen Geräts ist nun möglich. Die Entnahme kann beispielsweise durch eine erneute Krafteinwirkung entgegen der Rückstellkraft der Feder 3 und somit der Herstellung des Aufnahmezustands erleichtert werden.

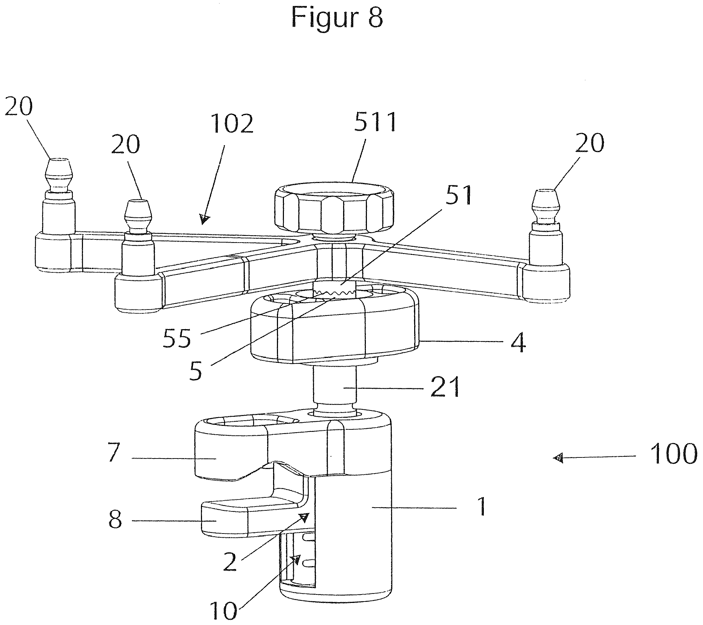

Die Figur 8 zeigt einen erfindungsgemäßen Adapter in einer zweiten Ausführungsform mit einem Lageerfassungssystem in Form eines Trackers mit drei Markierungselementen in einem Verklemmungszustand. Hinsichtlich der weiteren Beschreibungen wird auf die Beschreibungen der Figuren 2 bis 7 verwiesen.

### Bezugszeichenliste

1 erstes Halteelement
2 zweites Haltelement
3 Feder
4 zweites Verstellelement
5 Befestigungselement
6 Bodenfläche
7 erstes Klammerelement
8 zweites Klammerelement
10 Aufnahmeöffnung
1 1 erste Einfuhröffnung
12 zweite Einfuhröffnung
20 Markierungselement
21 erstes Verstellelement
51 Befestigungsgegenstück
55 Strukturelemente
101 medizintechnisches Gerät
102 Lageerfassungssystem
51 1 Mutter
F Rückstellkraft der Feder
V Verklemmrichtung
U Verschlussrichtung

## Patentansprüche

1. Adapter (100) zur Aufnahme eines medizintechnischen Geräts (101) sowie eines Lageerfassungssystems (102), umfassend
a. ein erstes Halteelement (1) und ein zweites Halteelement (2), wobei das erste Halteelement (1) eine Aufnahmeöffnung (10) mit einer Längserstreckungsrichtung aufweist, und wobei das zweite Halteelement (2) beweglich in der Aufnahmeöffnung des ersten Halteelements (1) gelagert ist;
b. eine Feder (3), welche zwischen dem zweiten Halteelement (2) und einer Bodenfläche (6), die an einer Unterseite des ersten Halteelements (1) festgelegt ist, angeordnet ist, wobei die Rückstellkraft der Feder (3) in der Längserstreckungsrichtung der Aufnahmeöffnung (10) des ersten Halteelements (1) verläuft und auf das zweite Halteelement (2) wirkt;
c. eine erste Einführöffnung (11), die das erste Halteelement (1) komplett durchdringt, und eine zweite Einführöffnung (12), die das zweite Halteelement (2) komplett durchdringt, beide zur Einführung des medizintechnischen Geräts (101), wobei die erste und zweite Einführöffnung (11, 12) quer zur Rückstellkraft der Feder (3) ausgebildet und durch eine Krafteinwirkung entgegen der Rückstellkraft der Feder (3) zur Deckung bringbar sind, wodurch ein Aufnahmezustand des Adapters (100) vorliegt, und wobei ohne die Krafteinwirkung entgegen der Rückstellkraft der Feder (3) ein Verklemmungszustand des Adapters (100) vorliegt;
d. ein erstes Verstellelement (21), welches an der Oberseite des zweiten Halteelements (2) festgelegt ist, und welches an seiner Außenhülle ein Gewinde aufweist;
e. ein zweites Verstellelement (4), welches über ein Gegengewinde mit dem Gewinde des ersten Verstellelements (21) verbunden ist, so dass sich mittels einer Drehbewegung des zweiten Verstellelements (4) das Gewinde des ersten Verstellelements (21) in Richtung der Rückstellkraft der Feder (3) in das zweite Verstellelement (4) bewegen lässt, bis dadurch ein bewegungssicherer Verklemmungszustand des Adapters (100) erreicht wird; und
f. ein Befestigungselement (5) zum Befestigen des Lageerfassungssystems (102).

2. Adapter (100) zur Aufnahme eines medizintechnischen Geräts (101) sowie eines Lageerfassungssystems (102), umfassend
a. ein erstes Halteelement (1) und ein zweites Halteelement (2), wobei das erste Halteelement (1) eine Aufnahmeöffnung (10) mit einer Längserstreckungsrichtung aufweist, und wobei das zweite Halteelement (2) beweglich in der Aufnahmeöffnung des ersten Halteelements (1) gelagert ist;
b. eine Feder (3), welche zwischen dem zweiten Halteelement (2) und einer Bodenfläche (6), die an einer Unterseite des ersten Halteelements (1) festgelegt ist, angeordnet ist, wobei die Rückstellkraft der Feder (3) in der Längserstreckungsrichtung der Aufnahmeöffnung (10) des ersten Halteelements (1) verläuft und auf das zweite Halteelement (2) wirkt;
c. ein erstes Klammerelement (7) des ersten Halteelements (1) und ein sich durch die Aufnahmeöffnung (10) nach außen hin erstreckendes zweites Klammerelement (8) des zweiten Halteelements (2), wobei durch eine Krafteinwirkung entgegen der Rückstellkraft der Feder (3) der Abstand zwischen dem ersten Klammerelement (7) und dem zweiten Klammerelement (8) vergrößert wird und der Aufnahmezustand des Adapters (100) vorliegt, und wobei bei Abwesenheit der Krafteinwirkung ein Verklemmungszustand des Adapters (100) vorliegt;
d. ein erstes Verstellelement (21), welches an der Oberseite des zweiten Halteelements (2) festgelegt ist, und welches an seiner Außenhülle ein Gewinde aufweist;
e. ein zweites Verstellelement (4), welches über ein Gegengewinde mit dem Gewinde des ersten Verstellelements (21) verbunden ist, so dass sich mittels einer Drehbewegung des zweiten Verstellelements (4) das Gewinde des ersten Verstellelements (21) in Richtung der Rückstellkraft der Feder (3) in das zweite Verstellelement (4) bewegen lässt, bis dadurch ein bewegungssicherer Verklemmungszustand des Adapters (100) erreicht wird; und
f. ein Befestigungselement (5) zum Befestigen des Lageerfassungssystems (102).

3. System umfassend einen Adapter nach einem der Ansprüche 1 oder 2 und ein Lageerfassungssystem, **dadurch gekennzeichnet, dass** das Lageerfassungssystem (102) ein Befestigungsgegenstück (51) aufweist, welches das Befestigungselement (5) in Eingriff nehmen kann.

4. System nach Anspruch 3, **dadurch gekennzeichnet, dass** das Befestigungselement (5) eine Aushöhlung mit einem Gewinde und das Befestigungsgegenstück (51) eine Aufnahmeöffnung mit einem identischen Gewinde aufweist, wobei die Aushöhlung des Befestigungselements (5) und die Aufnahmeöffnung des Befestigungsgegenstücks (51) in Richtung der Rückstellkraft der Feder (3) ausgerichtet sind und derart ausgebildet sind, dass mittels einer Schraube und einer Mutter (511) ein Verschlusszustand des Lageerfassungssystems (102) mit dem Adapter (100) durch eine kraftschlüssige Verbindung des Befestigungselements (5), des Befestigungsgegenstücks (51), der Schraube und der Mutter (511) über eine Drehbewegung der Mutter (511) in Verschlussrichtung herstellbar ist.

5. System nach Anspruch 3 oder 4, **dadurch gekennzeichnet, dass** das Befestigungselement (5) und das Befestigungsgegenstück (51) an ihren gegenseitigen Kontaktflächen ineinander eingreifende Strukturierungen (55), insbesondere ineinander eingreifende Zahnstrukturen, aufweisen, so dass im Verschlusszustand ein form- und kraftschlüssiger Verschluss des Lageerfassungssystems (102) mit dem Adapter (100) vorliegt.

6. System nach Anspruch 3 bis 5, **dadurch gekennzeichnet, dass** in einem nicht verschlossenen Zustand das Lageerfassungssystem (102) drehbar gelagert ist und somit die räumliche Ausrichtung des Lageerfassungssystems (102) variabel einstellbar ist.

7. System nach Anspruch 6, **dadurch gekennzeichnet, dass** die räumliche Anordnung des Lageerfassungssystems (102) in einem Winkel einstellbar ist, der durch die Größe der Strukturelemente (55) des Befestigungselements (5) und des Befestigungsgegenstücks (51) vorgegebenen ist.

8. Verfahren zur Aufnahme eines medizintechnischen Geräts (101) sowie eines Lageerfassungssystems (102) durch einen Adapter (100), umfassend die Schritte:
a. Bereitstellen eines Adapters nach Anspruch 1;
b. Herstellen des Aufnahmezustands durch Ausüben einer Kraft entgegen der Rückstellkraft der Feder (3);
c. Einführen des medizintechnischen Geräts in die erste Einführöffnung (11) des ersten Halteelements (1) und die zweite Einführöffnung (12) des zweiten Halteelements (2);
d. Herstellen des bewegungssicheren Verklemmungszustands durch Drehbewegung des zweiten Verstellelements (4);
e. Befestigung des Lageerfassungssystems (102) an dem Befestigungselement (5).

9. Verwendung eines Adapters (100) nach Anspruch 1 zur Aufnahme eines medizintechnischen Geräts (101) sowie eines Lageerfassungssystems.

10. Verfahren zur Aufnahme eines medizintechnischen Geräts (101) sowie eines Lageerfassungssystems (102) durch einen Adapter (100), umfassend die Schritte:
a. Bereitstellen eines Adapters nach Anspruch 2;
b. Herstellen des Aufnahmezustands durch Ausüben einer Kraft entgegen der Rückstellkraft der Feder (3);
c. Einführen des medizintechnischen Geräts in das erste Klammerelement (7) des ersten Halteelements (1) und das zweite Klammerelement (8) des zweiten Halteelements (2);
d. Herstellen des bewegungssicheren Verklemmungszustands durch Drehbewegung des zweiten Verstellelements (4);
e. Befestigung des Lageerfassungssystems (102) an dem Befestigungselement (5).

11. Verwendung eines Adapters (100) nach Anspruch 2 zur Aufnahme eines medizintechnischen Geräts (101) sowie eines Lageerfassungssystems.

## Claims

1. An adapter (100) for receiving a medical technical device (101) and a position detecting system (102), comprising:
a. a first holding element (1) and a second holding element (2), wherein the first holding element (1) has a receiving opening (10) with a longitudinal extension direction, and wherein the second holding element (2) is movably supported in the receiving opening of the first holding element (1);
b. a spring (3) disposed between the second holding element (2) and a bottom surface (6) fixed at a bottom side of the first holding element (1), wherein the restoring force of the spring (3) is directed in the longitudinal extension direction of the first holding element (1) and acts on the second holding element (2);
c. a first insertion opening (11) completely passing through the first holding element (1) and a second insertion opening (12) completely passing through the second holding element (2), both for insertion of the medical technical device (101), wherein the first and second insertion openings (11, 12) are configured transverse to the restoring force of the spring (3) and can be brought into congruence by a force acting counter to the restoring force of the spring (3), whereby a receiving state of the adapter (100) is defined, and wherein without the force acting counter to the restoring force of the spring (3), a clamping state of the adapter (100) is defined;
d. a first displacing element (21) fixed at the top side of the second holding element (2) and having a thread on its outer face;
e. a second displacing element (4) connected to the thread of the first displacing element (21) via a counter-thread, such that by a rotational movement of the second displacing element (4) the thread of the first displacing element (21) can be moved in the direction of the restoring force of the spring (3) into the second displacing element (4), until a movement proof clamping state of the adapter (100) is thereby achieved; and
f. a fixing element (5) for fixing the position detecting system (102).

2. An adapter (100) for receiving a medical technical device (101) and a position detecting system (102), comprising:
a. a first holding element (1) and a second holding element (2), wherein the first holding element (1) has a receiving opening (10) with a longitudinal extension direction, and wherein the second holding element (2) is movably supported in the receiving opening of the first holding element (1);
b. a spring (3) disposed between the second holding element (2) and a bottom surface (6) fixed at a bottom side of the first holding element (1), wherein the restoring force of the spring (3) is directed in the longitudinal extension direction of the first holding element (1) and acts on the second holding element (2);
c. a first clamping element (7) of the first holding element (1) and a second clamping element (8) of the second holding element (2) extending outwards through the receiving opening (10), wherein by a force acting counter the restoring force of the spring (3) the distance between the first clamping element (7) and the second clamping element (8) is increased and the receiving state of the adapter (100) is defined, and wherein in the absence of the acting force the clamping state of the adapter (100) is defined;
d. a first displacing element (21) fixed at the top side of the second holding element (2) and having a thread on its outer face;
e. a second displacing element (4) connected to the thread of the first displacing element (21) via a counter-thread, such that by a rotational movement of the second displacing element (4) the thread of the first displacing element (21) can be moved in the direction of the restoring force of the spring (3) into the second displacing element (4), until a movement proof clamping state of the adapter (100) is thereby achieved; and
f. a fixing element (5) for fixing the position detecting system (102).

3. A system comprising an adapter according to any one of claims 1 to 2, **characterized in that** the position detecting system (102) comprises an fixing counter-element (51) which can engage the fixing element (5).

4. The system according to claim 3, **characterized in that** the fixing element (5) has a cavity with a thread and the fixing counter-element (51) has a receiving opening with an identical thread, wherein the cavity of the fixing element (5) and the receiving opening of the fixing counter-element (51) are aligned in the direction of the restoring force of the spring (3) and are configured so that by means of a screw and a nut (511), a locking state of the position detecting system (102) with the adapter (100) can be obtained by a force-fitting connection of the fixing element (5), the fixing counter-element (51), the screw and the nut (511) by means of a rotational movement of the nut (511) in a locking direction.

5. The system according to claim 3 or 4, **characterized in that** the fixing element (5) and the fixing counter-element (51) have engaging structures (55), in particular engaging tooth structures, on their mutual contact surfaces, so that in the locking state a form- and force-fitted locking of the position detecting system (102) with the adapter (100) is defined.

6. The system according to any one of claims 3 to 5, **characterized in that** in an unlocked state the position detecting system (102) is rotatably supported and thus the spatial orientation of the position detecting system (102) is variably adjustable.

7. The system according to claim 6, **characterized in that** the spatial positioning of the position detecting system (102) is adjustable at an angle that is predetermined by the size of the structure elements (55) of the fixing element (5) and the fixing counter-element (51).

8. A method for receiving a medical technical device (101) and a position detecting system (102) by an adapter (100), comprising the steps:
a. providing an adapter according to claim 1;
b. establishing the receiving state by applying a force against the restoring force of the spring (3);
c. inserting the medical device in the first insertion opening (11) of the first holding element (1) and the second insertion opening (12) of the second holding element (2);
d. establishing the movement proof clamping state by rotational movement of the second displacing element (4);
e. fixing of the position detecting system (102) to the fixing element (5).

9. Use of an adapter (100) according to claim 1 for receiving a medical technical device (101) and a position detecting system.

10. A method for receiving a medical technical device (101) and a position detecting system (102) by an adapter (100), comprising the steps:
a. providing an adapter according to claim 2;
b. establishing the receiving state by applying a force against the restoring force of the spring (3);
c. inserting the medical device in the first clamping element (7) of the first holding element (1) and the second clamping element (8) of the second holding element (2);
d. establishing the movement proof clamping state by rotational movement of the second displacing element (4);
e. fixing of the position detecting system (102) to the fixing element (5).

11. Use of an adapter (100) according to claim 2 for receiving a medical technical device (101) and a position detecting system.

## Revendications

1. Adaptateur (100) destiné à recevoir un appareil médical (101) et un système de détection de position (102), cet adaptateur comprenant
a) un premier élément support (1) et un deuxième élément support (2), le premier élément support (1) présentant une ouverture de réception (10) ayant une direction d'extension longitudinale, et le deuxième élément support (2) étant logé mobile dans l'ouverture de réception du premier élément support (1) ;
b) un ressort (3) lequel est disposé entre le deuxième élément support (2) et une surface de fond (6) fixée sur la face inférieure du premier élément support (1), la force de rappel du ressort (3) s'étendant dans la direction d'extension longitudinale de l'ouverture de réception (10) du premier élément support (1) et s'exerçant sur le deuxième élément support (2) ;
c) une première ouverture d'introduction (11) qui traverse complètement le premier élément support (1) et une deuxième ouverture d'introduction (12) qui traverse complètement le deuxième élément support (2), ces deux ouvertures servant à l'introduction de l'appareil médical (101), la première et la deuxième ouverture d'introduction (11, 12) étant conçues perpendiculairement à la force de rappel du ressort (3) et pouvant, sous l'effet d'une force antagoniste à la force de rappel du ressort (3), être amenées en recouvrement pour obtenir un état de réception de l'adaptateur (100), et l'absence d'une force antagoniste à la force de rappel du ressort (3) permettant d'obtenir un état de blocage de l'adaptateur (100) ;
d) un premier élément de positionnement (21) lequel est fixé sur la face supérieure du deuxième élément support (2) et présente un filetage sur son enveloppe extérieure ;
e) un deuxième élément de positionnement (4) lequel est relié au filetage du premier élément de positionnement (21) par le biais d'un contre-filetage si bien que le filetage du premier élément de positionnement (21) peut, par mouvement rotatif du deuxième élément de positionnement (4), être déplacé dans le sens de la force de rappel du ressort (3) dans le deuxième élément de positionnement (4) jusqu'à atteinte d'un état de blocage de l'adaptateur (100) maintenu ainsi en position fixe et sécurisé contre tout mouvement ;
f) un élément de fixation (5) pour fixer le système de détection de position (102).

2. Adaptateur (100) destiné à recevoir un appareil médical (101) et un système de détection de position (102), cet adaptateur comprenant :
a) un premier élément support (1) et un deuxième élément support (2), le premier élément support (1) présentant une ouverture de réception (10) ayant une direction d'extension longitudinale, et le deuxième élément support (2) étant logé mobile dans l'ouverture de réception du premier élément support (1) ;
b) un ressort (3) lequel est disposé entre le deuxième élément support (2) et une surface de fond (6) fixée sur la face inférieure du première élément support (1), la force de rappel du ressort (3) s'étendant dans la direction d'extension longitudinale de l'ouverture de réception (10) du premier élément support (1) et s'exerçant sur le deuxième élément support (2) ;
c) un premier élément de serrage (7) du premier élément support (1) et un deuxième élément de serrage (8) du deuxième élément support (2) s'étendant vers l'extérieur à travers l'ouverture de réception (10), la distance entre le premier élément de serrage (7) et le deuxième élément de serrage (8) s'agrandissant sous l'effet d'une force antagoniste à la force de rappel du ressort (3) pour obtenir l'état de réception de l'adaptateur (100), et l'absence de la force antagoniste permettant d'obtenir un état de blocage de l'adaptateur (100) ;
d) un premier élément de positionnement (21) lequel est fixé sur la face supérieure du deuxième élément support (2) et présente un filetage sur son enveloppe extérieure ;
e) un deuxième élément de positionnement (4) qui est relié au filetage du premier élément de positionnement (21) par le biais d'un contre-filetage si bien que le filetage du premier élément de positionnement (21) peut, par mouvement rotatif du deuxième élément de positionnement (4), être déplacé dans le sens de la force de rappel du ressort (3) dans le deuxième élément de positionnement (4) jusqu'à atteinte d'un état de blocage de l'adaptateur (100) maintenu ainsi en position fixe et sécurisé contre tout mouvement ;
f) un élément de fixation (5) pour fixer le système de détection de position (102).

3. Système comprenant un adaptateur selon la revendication 1 ou 2 et un système de détection de position, **caractérisé en ce que** le système de détection de position (102) présente un contre-élément de fixation (51) pouvant venir en prise avec l'élément de fixation (5).

4. Système selon la revendication 3, **caractérisé en ce que** l'élément de fixation (5) présente un évidement muni d'un filetage, et le contre-élément de fixation (51) une ouverture de réception munie d'un filetage identique, l'évidement de l'élément de fixation (5) et l'ouverture de réception du contre-élément de fixation (51) étant orientés dans le sens de la force de rappel du ressort (3) et conçus de telle sorte qu'au moyen d'une vis et d'un écrou (511), un état de fermeture du système de détection de position (102) avec l'adaptateur (100) peut être obtenu par mouvement rotatif de l'écrou dans le sens de fermeture à l'aide d'une liaison de force entre l'élément de fixation (5), le contre-élément de fixation (51), la vis et l'écrou (511).

5. Système selon la revendication 3 ou 4, **caractérisé en ce que** l'élément de fixation (5) et le contre-élément de fixation (51) présentent sur leurs faces de contact réciproques des structures (55) venant en prise mutuelle, plus particulièrement des structures dentées venant en prise mutuelle si bien qu'à l'état de fermeture, il est obtenu une fermeture par force et par correspondance de forme du système de détection de position (102) avec l'adaptateur (100).

6. Système selon les revendications 3 à 5, **caractérisé en ce que** dans un état non fermé, le système de détection de position (102) est logé rotatif en faveur d'un réglage variable de l'orientation dans l'espace du système de détection de position (102).

7. Système selon la revendication 6, **caractérisé en ce que** la disposition dans l'espace du système de détection de position (102) est réglable selon un angle qui est prédéfini par la taille des éléments de structure (55) de l'élément de fixation (5) et du contre-élément de fixation (51).

8. Procédé permettant l'installation d'un appareil médical (101) et d'un système de détection de position (102) à l'aide d'un adaptateur (100), ce procédé comprenant les étapes suivantes :
a) la mise à disposition d'un adaptateur selon la revendication 1 ;
b) la mise en oeuvre de l'état de réception par l'exercice d'une force antagoniste à la force de rappel du ressort (3) ;
c) l'introduction de l'appareil médical dans la première ouverture d'introduction (11) du premier élément support (1) et dans la deuxième ouverture d'introduction (12) du deuxième élément support (2) ;
d) la mise en oeuvre de l'état de blocage pour maintien en position fixe et sécurisation contre tout mouvement ;
e) la fixation du système de détection de position (102) sur l'élément de fixation (5) ;

9. Utilisation d'un adaptateur (100) selon la revendication 1 destiné à recevoir un appareil médical (101) et un système de détection de position.

10. Procédé permettant l'installation d'un appareil médical (101) et d'un système de détection de position (102) à l'aide d'un adaptateur (100), ce procédé comprenant les étapes suivantes :
a) la mise à disposition d'un adaptateur selon la revendication 2 ;
b) la mise en oeuvre de l'état de réception par l'exercice d'une force antagoniste à la force de rappel du ressort (3) ;
c) l'introduction de l'appareil médical dans le premier élément de serrage (7) du premier élément support (1) et dans le deuxième élément de serrage (8) du deuxième élément support (2) ;
d) la mise en oeuvre de l'état de blocage pour maintien en position fixe et sécurisation contre tout mouvement par mouvement rotatif du deuxième élément de positionnement (4) ;
e) la fixation du système de détection de position (102) sur l'élément de fixation (5) ;

11. Utilisation d'un adaptateur (100) selon la revendication 2 destiné à recevoir un appareil médical (101) et un système de détection de position.
